# EUROPEAN PATENT APPLICATION

(11) **EP 4 660 292 A1**
(43) Date of publication of application: **10.12.2025**
(21) Application number: 24763949.5
(22) Date of filing: 28.02.2024
(51) Int. Cl.: C12N 1/00, C12M 1/02, C12Q 1/02

(54) **STIRRING METHOD AND STIRRING DEVICE**

(30) Priority: 28.02.2023 JP 2023029017
(71) Applicant: Terumo Kabushiki Kaisha, Tokyo 151-0072 (JP)
(72) Inventor: LIU, Liming, Ashigarakami-gun, Kanagawa 259-0151 (JP); OKUBO, Itaru, Ashigarakami-gun, Kanagawa 259-0151 (JP)
(74) Representative: TBK
(86) International application number: PCT/JP2024/007196
(87) International publication number: WO 2024/181470

(57) **Abstract**

A stirring method includes: a cell stirring step of stirring cell suspension by circulating the cell suspension in a circulation path (130) including a bioreactor (24); an acquisition step of acquiring a predetermined physical quantity related to the cell suspension at a measurement target site in the circulation path (130) over time; and a determination step of determining that a uniform diffusion state in which cells are uniformly diffused in the cell suspension is reached on the basis of a fact that a degree of variation of the physical quantity is smaller than a predetermined degree.

## Description

### Technical Field

The present invention relates to a stirring method and a stirring apparatus for stirring a cell suspension.

### Background Art

In order to efficiently culture the cells, it is preferable to appropriately stir the cell suspension during cell culture. By stirring the cell suspension, all cells in the cell suspension can be nourished. Japanese Patent Application Laid-Open No. 2002-148258 discloses a ventilation stirring type culture apparatus.

### Summary of Invention

In the cell culture processing, a series of processing including a cell stirring step and a cell culture step is repeatedly performed, for example, every 24 hours. In order to optimize the cell culture processing, a technique for optimizing the cell stirring step is awaited.

An object of the present invention is to solve the above-described problem.
(1) A stirring method according to a first aspect of the present invention includes: a cell stirring step of stirring cell suspension by circulating the cell suspension in a circulation path including a bioreactor; an acquisition step of acquiring a predetermined physical quantity related to the cell suspension at a measurement target site in the circulation path over time; and a determination step of determining that a uniform diffusion state in which cells are uniformly diffused in the cell suspension is reached on the basis of a fact that a degree of variation of the physical quantity is smaller than a predetermined degree.
   In the first aspect of the present invention, in the determination step, the degree of variation is compared with a predetermined degree. As a result, in the determination step, it is possible to determine whether or not the uniform diffusion state has been reached. According to the first aspect of the present invention, the cell stirring step can be performed only for a necessary minimum time. According to the first aspect of the present invention, the execution time of the cell stirring step can be shortened, and the execution time of the cell culture processing can be shortened. Therefore, according to the first aspect of the present invention, it is possible to contribute to optimization of cell culture processing.
(2) In the stirring method according to the above item (1), the degree of variation of the physical quantity may be grasped on the basis of a standard deviation of a plurality of the physical quantity within a predetermined unit time.
(3) In the stirring method according to the above items (1) or (2), the physical quantity may be acquired using an optical sensor.
(4) In the stirring method according to any one of the above items (1) to (3), the physical quantity may be at least one of an amount of transmitted light transmitted through the cell suspension and an amount of scattered light scattered by the cell suspension.
(5) In the stirring method according to any one of the above items (1) to (4), in the cell stirring step, stirring of the cell suspension may be stopped in a case where it is determined in the determination step that the uniform diffusion state is reached.
(6) In the stirring method according to any one of the above items (1) to (5), in the cell stirring step, the cell suspension may be stirred by rotating the bioreactor using an actuator.
   According to the configuration of the above item (6), the cell suspension can be brought into a uniform diffusion state more quickly.
(7) A stirring apparatus according to a second aspect of the present invention includes: a control unit that stirs cell suspension by circulating the cell suspension in a circulation path including a bioreactor; an acquisition unit that acquires a predetermined physical quantity related to the cell suspension at a measurement target site in the circulation path over time; and a determination unit that determines that a uniform diffusion state in which cells are uniformly diffused in the cell suspension is reached on the basis of a fact that a degree of variation of the physical quantity is smaller than a predetermined degree.
   According to the second aspect of the present invention, an effect equivalent to that of the first aspect of the present invention can be obtained.
(8) In the stirring apparatus according to the above item (7), the degree of variation of the physical quantity may be grasped on the basis of a standard deviation of a plurality of the physical quantity within a predetermined unit time.
(9) In the stirring apparatus according to the above items (7) or (8), the physical quantity may be acquired using an optical sensor.
(10) In the stirring apparatus according to any one of the above items (7) to (9), the physical quantity may be at least one of an amount of transmitted light transmitted through the cell suspension and an amount of scattered light scattered by the cell suspension.
(11) In the stirring apparatus according to any one of the above items (7) to (10), the control unit may stop stirring of the cell suspension in a case where the determination unit determines that the uniform diffusion state is reached.
(12) In the stirring apparatus according to any one of the above items (7) to (11), the control unit may stir the cell suspension by rotating the bioreactor using an actuator.

According to the configuration of the above item (12), an effect equivalent to that of the above item (6) can be obtained.

According to the present invention, it is possible to contribute to the optimization of the cell stirring step.

### Brief Description of Drawings

Fig. 1 is a schematic diagram illustrating a cell culture apparatus.
Fig. 2 is a block diagram of a culture control apparatus (stirring apparatus) included in the cell culture apparatus.
Fig. 3 is a flowchart of cell culture processing.
Fig. 4 is a schematic diagram illustrating a cell loading step.
Fig. 5 is a schematic diagram illustrating a cell stirring step.
Fig. 6 is a schematic diagram illustrating a cell packing step.
Fig. 7 is a schematic diagram illustrating a cell culture step.
Fig. 8 is a flowchart of a cell stirring step.
Figs. 9A and 9B are time charts illustrating a temporal change in the amount of received transmitted light detected by a sensor unit.

### Description of Embodiments

### [1 Configuration of Cell Culture Apparatus 10]

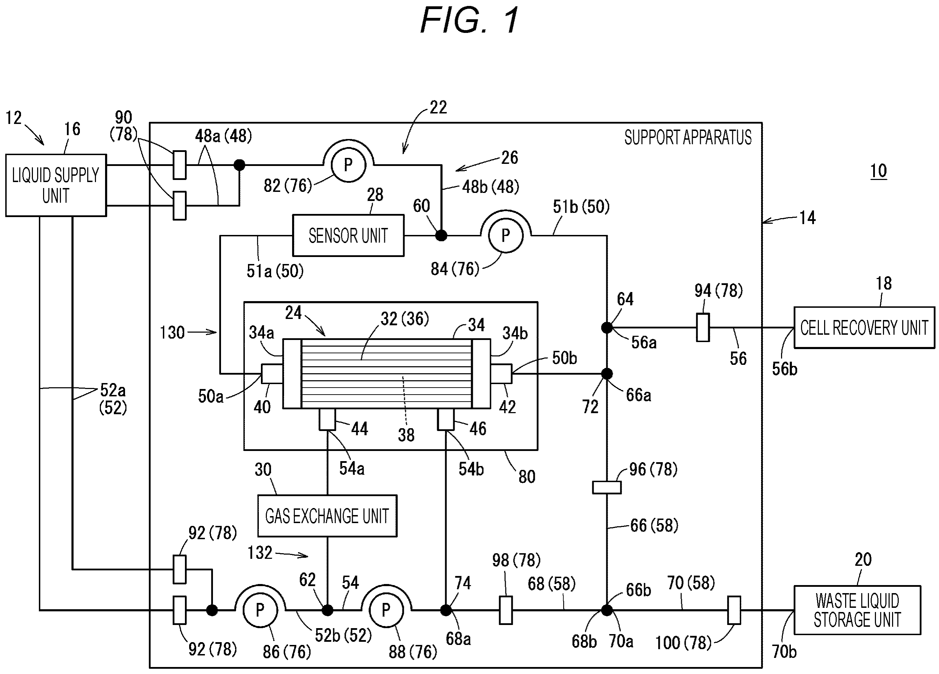

Fig. 1 is a schematic diagram illustrating a cell culture apparatus 10. The cell culture apparatus 10 includes a cell culture circuit 12 and a support apparatus 14. The cell culture apparatus 10 cultures the cells separated from the biological tissue in a culture medium. The cell cultured in the cell culture apparatus 10 is, for example, an adherent cell or a floating cell. Examples of the cells cultured in the cell culture apparatus 10 include ES cells, iPS cells, and mesenchymal stem cells. Note that the cells cultured by the cell culture apparatus 10 are not limited to the above-described cells.

### [1-1 Cell Culture Circuit 12]

Liquid flows through a cell culture circuit 12. Examples of the liquid include a cell suspension, a culture medium, a washing solution, and a dissociation solution. The cell suspension is a solution containing cells. The culture medium is a culture solution for growing cells. The culture medium is selected according to the cell to be cultured. As the culture medium, for example, minimum essential media (MEM) are used. The washing solution is a solution for washing the inside of the cell culture circuit 12. Examples of the washing solution include water, a buffer solution, and physiological saline. Examples of the buffer solution include phosphate buffered salts (PBS), tris-buffered saline (TBS), and the like. The dissociation solution is a solution for detaching cells from a bioreactor 24 of the cell culture circuit 12. Examples of the dissociation solution include a trypsin solution and an EDTA solution (ethylenediaminetetraacetic acid solution). Note that the culture medium, the washing solution, and the dissociation solution are not limited to the liquid described above.

The cell culture circuit 12 is a disposable product that is replaced for each cell culture processing. The cell culture circuit 12 includes a liquid supply unit 16, a cell recovery unit 18, a waste liquid storage unit 20, and a culture body 22.

The liquid supply unit 16 includes a plurality of medical bags (not illustrated). Each medical bag is filled with a liquid to be supplied to the culture body 22. For example, the first medical bag is filled with a cell suspension. The second medical bag is filled with a culture medium. The third medical bag is filled with a washing solution. The fourth medical bag is filled with a dissociation solution. Note that each of the plurality of types of culture media may be individually filled in the medical bag.

Each of the cell recovery unit 18 and the waste liquid storage unit 20 includes a medical bag (not illustrated). The cell recovery unit 18 recovers the cells cultured in the culture body 22. The waste liquid storage unit 20 stores the waste liquid generated in the culture body 22.

The culture body 22 includes a bioreactor 24, a flow path 26, a sensor unit 28, and a gas exchange unit 30.

The bioreactor 24 comprises a plurality of hollow fiber membranes 32 and a cylindrical housing 34. The plurality of hollow fiber membranes 32 is stored in the housing 34. Each hollow fiber membrane 32 extends along the longitudinal direction of the bioreactor 24. The hollow fiber membrane 32 is made of, for example, a polymer material. The hollow fiber membrane 32 has a plurality of pores (not illustrated). A first end portion of each hollow fiber membrane 32 is fixed to a first end portion 34a in the longitudinal direction of the housing 34. The second end portion of each hollow fiber membrane 32 is fixed to the second end portion 34b in the longitudinal direction of the housing 34.

The bioreactor 24 comprises a first region 36 and a second region 38. The first region 36 is a space inside each hollow fiber membrane 32. The second region 38 is a space between the outer peripheral surface of each hollow fiber membrane 32 and the inner peripheral surface of the housing 34. The first region 36 and the second region 38 communicate with each other through the plurality of pores of each hollow fiber membrane 32.

The housing 34 includes a first port 40, a second port 42, a third port 44, and a fourth port 46. The first port 40 is disposed at the first end portion 34a of the housing 34. The first port 40 is connected to a first end portion of each hollow fiber membrane 32. As a result, the first port 40 communicates with the first region 36. The second port 42 is disposed at the second end portion 34b of the housing 34. The second port 42 is connected to the second end portion of each hollow fiber membrane 32. As a result, the second port 42 communicates with the first region 36.

The third port 44 and the fourth port 46 are disposed on the outer peripheral surface of the housing 34. The third port 44 is disposed between the first port 40 and a central portion of the housing 34 in the longitudinal direction. The fourth port 46 is disposed between the second port 42 and a central portion of the housing 34 in the longitudinal direction. Each of the third port 44 and the fourth port 46 communicates with the second region 38.

The flow path 26 includes a plurality of tubes (pipes) through which liquid flows. Each tube is made of a soft resin material. The flow path 26 includes a first supply flow path 48, a first circulation flow path 50, a second supply flow path 52, a second circulation flow path 54, a collection flow path 56, and a waste liquid flow path 58.

The first supply flow path 48 introduces various liquids of the liquid supply unit 16 into the first circulation flow path 50. The first supply flow path 48 includes a plurality of first upstream flow paths 48a and one first downstream flow path 48b. One first upstream flow path 48a is provided for one medical bag of the liquid supply unit 16. The first upstream flow path 48a is connected to the medical bag of the liquid supply unit 16. In addition, each of the first upstream flow paths 48a is connected to the first downstream flow path 48b. The first downstream flow path 48b is connected to a first merging portion 60 of the first circulation flow path 50.

The first circulation flow path 50 introduces the liquid introduced from the first supply flow path 48 into the bioreactor 24. In addition, the first circulation flow path 50 introduces the liquid discharged from the bioreactor 24 into the bioreactor 24 again. The first end portion 50a of the first circulation flow path 50 is connected to the first port 40 of the bioreactor 24. The second end portion 50b of the first circulation flow path 50 is connected to the second port 42 of the bioreactor 24. The first circulation flow path 50 communicates with an inner hole (first region 36) of each hollow fiber membrane 32. The first merging portion 60 is disposed in the first circulation flow path 50. In the first circulation flow path 50, a portion partitioned by the first end portion 50a and the first merging portion 60 is referred to as a first flow path 51a. In the first circulation flow path 50, a portion partitioned by the second end portion 50b and the first merging portion 60 is referred to as a second flow path 51b. A collection branch portion 64 is disposed in the second flow path 51b. Furthermore, in the second flow path 51b, a first branch portion 72 is disposed between the collection branch portion 64 and the second end portion 50b.

The second supply flow path 52 introduces various liquids of the liquid supply unit 16 into the second circulation flow path 54. The second supply flow path 52 includes a plurality of second upstream flow paths 52a and one second downstream flow path 52b. One second upstream flow path 52a is provided for one medical bag of the liquid supply unit 16. The second upstream flow path 52a is connected to the medical bag of the liquid supply unit 16. In addition, each of the second upstream flow paths 52a is connected to the second downstream flow path 52b. The second downstream flow path 52b is connected to a second merging portion 62 of the second circulation flow path 54.

The second circulation flow path 54 introduces the liquid introduced from the second supply flow path 52 into the bioreactor 24. In addition, the second circulation flow path 54 introduces the liquid discharged from the bioreactor 24 into the bioreactor 24 again. The first end portion 54a of the second circulation flow path 54 is connected to the third port 44 of the bioreactor 24. A second end portion 54b of the second circulation flow path 54 is connected to the fourth port 46 of the bioreactor 24. The second circulation flow path 54 communicates with a space (second region 38) between the plurality of hollow fiber membranes 32 and the housing 34. The second merging portion 62 is disposed in the second circulation flow path 54. In addition, in the second circulation flow path 54, a second branch portion 74 is disposed between the second merging portion 62 and the second end portion 54b.

The collection flow path 56 introduces the cell suspension discharged from the bioreactor 24 into the cell recovery unit 18. The collection flow path 56 branches from the first circulation flow path 50. A first end portion 56a of the collection flow path 56 is connected to the collection branch portion 64 of the first circulation flow path 50. A second end portion 56b of the collection flow path 56 is connected to the medical bag of the cell recovery unit 18.

The waste liquid flow path 58 introduces the liquid in the first circulation flow path 50 and the second circulation flow path 54 into the waste liquid storage unit 20. The waste liquid flow path 58 includes a first waste liquid flow path 66, a second waste liquid flow path 68, and a third waste liquid flow path 70. The first waste liquid flow path 66 branches from the first circulation flow path 50. A first end portion 66a of the first waste liquid flow path 66 is connected to the first branch portion 72 of the first circulation flow path 50. The second waste liquid flow path 68 branches from the second circulation flow path 54. A first end portion 68a of the second waste liquid flow path 68 is connected to the second branch portion 74 of the second circulation flow path 54. Each of the second end portion 66b of the first waste liquid flow path 66 and the second end portion 68b of the second waste liquid flow path 68 is connected to a first end portion 70a of the third waste liquid flow path 70. A second end portion 70b of the third waste liquid flow path 70 is connected to the medical bag of the waste liquid storage unit 20.

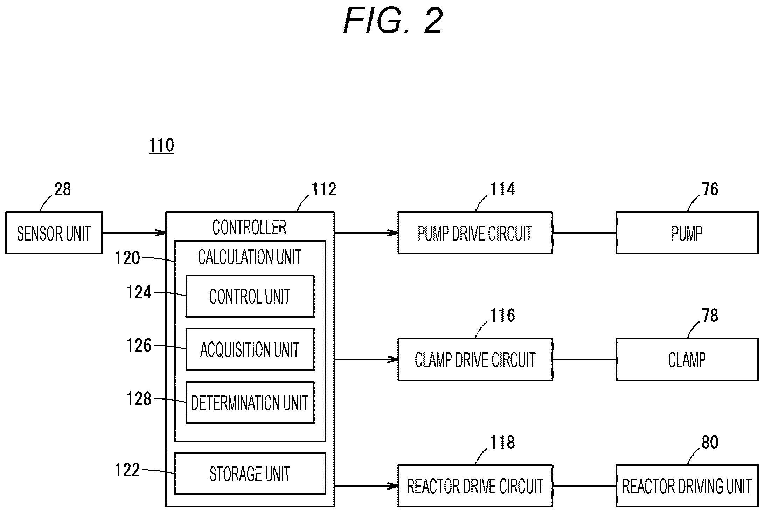

The sensor unit 28 is disposed in the first flow path 51a (measurement target site). Note that the sensor unit 28 may be disposed in the second flow path 51b. The sensor unit 28 detects a predetermined physical quantity related to a liquid (for example, a cell suspension) flowing through the first circulation flow path 50. The predetermined physical quantity is a physical quantity proportional to the number of cells in the liquid. For example, the sensor unit 28 includes a light source and one or more optical sensors (light receivers). The light source irradiates the liquid with light. The optical sensor receives transmitted light transmitted through the liquid. The optical sensor outputs an electric signal corresponding to the amount of received light to a controller 112 (Fig. 2). Note that the optical sensor may receive scattered light (forward scattered light, side scattered light, backscattered light, and the like) instead of the transmitted light. Furthermore, the sensor unit 28 may include a sensor (for example, a dielectric constant sensor) including two or more electrodes instead of the light source and the optical sensor.

The gas exchange unit 30 is disposed between the second merging portion 62 and the third port 44 in the second circulation flow path 54. The gas exchange unit 30 supplies a gas of a predetermined component to the liquid (culture medium) flowing through the second circulation flow path 54. The gas used in the gas exchange unit 30 has, for example, a component close to air. That is, the gas contains nitrogen, oxygen, and carbon dioxide.

### [1-2 Support Apparatus 14]

The cell culture circuit 12 described above is detachable from the support apparatus 14. The support apparatus 14 includes a cassette that supports the cell culture circuit 12. The support apparatus 14 is a reusable product that can be used a plurality of times.

The support apparatus 14 includes a plurality of pumps 76, a plurality of clamps 78, and a reactor driving unit 80. Each of the plurality of pumps 76, the plurality of clamps 78, and the reactor driving unit 80 includes an electric actuator. Note that each of the plurality of pumps 76, the plurality of clamps 78, and the reactor driving unit 80 may include a fluid actuator.

Each pump 76 can apply a flow force to the liquid in the flow path 26 by squeezing the tube forming the flow path 26. Each pump 76 is operated by power supplied from a pump drive circuit 114 (Fig. 2).

The plurality of pumps 76 includes a first supply pump 82, a first circulation pump 84, a second supply pump 86, and a second circulation pump 88. Each pump 76 functions as follows. Note that, as illustrated in Fig. 1, a state in which the cell culture circuit 12 is attached to the support apparatus 14 is referred to as a "set state".

In the set state, the first downstream flow path 48b is attached to the first supply pump 82. The first supply pump 82 applies a flow force in a direction from the liquid supply unit 16 toward the first circulation flow path 50 to the liquid in the first supply flow path 48.

In the set state, the second flow path 51b of the first circulation flow path 50 is attached to the first circulation pump 84. The first circulation pump 84 applies a flow force in a direction from the second port 42 to the first port 40 to the liquid in the first circulation flow path 50. Note that the first circulation pump 84 can also apply a flow force in a direction from the first port 40 to the second port 42 to the liquid in the first circulation flow path 50.

In the set state, the second downstream flow path 52b is attached to the second supply pump 86. The second supply pump 86 applies a flow force in a direction from the liquid supply unit 16 toward the second circulation flow path 54 to the liquid in the second supply flow path 52.

In the set state, the second circulation flow path 54 is attached to the second circulation pump 88. The second circulation pump 88 applies a flow force in a direction from the fourth port 46 to the third port 44 to the liquid in the second circulation flow path 54. Note that the second circulation pump 88 can also apply a flow force in a direction from the third port 44 to the fourth port 46 to the liquid in the second circulation flow path 54.

Each clamp 78 can close the flow path 26 by compressing the tube forming the flow path 26 in the lateral direction. Each clamp 78 functions as an on-off valve. Each clamp 78 is operated by power supplied from a clamp drive circuit 116 (Fig. 2).

The plurality of clamps 78 includes a plurality of first supply clamps 90, a plurality of second supply clamps 92, a collection clamp 94, a first waste liquid clamp 96, a second waste liquid clamp 98, and a third waste liquid clamp 100. Each clamp 78 functions as follows.

In the set state, one first upstream flow path 48a is attached to one first supply clamp 90. In other words, each of the first upstream flow paths 48a is supported by any one of the first supply clamps 90. The first supply clamp 90 opens and closes the first supply flow path 48.

In the set state, one second upstream flow path 52a is attached to one second supply clamp 92. In other words, each of the second upstream flow paths 52a is supported by any one of the second supply clamps 92. The second supply clamp 92 opens and closes the second supply flow path 52.

In the set state, the collection flow path 56 is attached to the collection clamp 94. The collection clamp 94 opens and closes the collection flow path 56. In the set state, the first waste liquid flow path 66 is attached to the first waste liquid clamp 96. The first waste liquid clamp 96 opens and closes the first waste liquid flow path 66. In the set state, the second waste liquid flow path 68 is attached to the second waste liquid clamp 98. The second waste liquid clamp 98 opens and closes the second waste liquid flow path 68. In the set state, the third waste liquid flow path 70 is attached to the third waste liquid clamp 100. The third waste liquid clamp 100 opens and closes the third waste liquid flow path 70.

The reactor driving unit 80 supports the bioreactor 24. In addition, the reactor driving unit 80 can rotate the bioreactor 24 in one direction and the opposite direction about an axis orthogonal to the longitudinal direction of the bioreactor 24. The reactor driving unit 80 is operated by power supplied from a reactor drive circuit 118 (Fig. 2).

### [1-3 Culture Control Apparatus (Stirring Apparatus) 110]

Fig. 2 is a block diagram of a culture control apparatus 110 included in the cell culture apparatus 10. The culture control apparatus 110 controls each of the plurality of pumps 76, the plurality of clamps 78, and the reactor driving unit 80. Note that the culture control apparatus 110 functions as a stirring apparatus that stirs the cell suspension of the bioreactor 24.

The culture control apparatus 110 includes a sensor unit 28, a controller 112, a pump drive circuit 114, a clamp drive circuit 116, and a reactor drive circuit 118. As the controller 112, for example, a computer is used. The controller 112 includes a calculation unit 120 and a storage unit 122.

The calculation unit 120 can be configured by, for example, a processor such as a central processing unit (CPU) or a graphics processing unit (GPU). That is, the calculation unit 120 can be configured by a processing circuit.

The calculation unit 120 includes a control unit 124, an acquisition unit 126, and a determination unit 128. Each of the control unit 124, the acquisition unit 126, and the determination unit 128 can be realized by executing a program stored in the storage unit 122 by the calculation unit 120.

Note that at least a part of the control unit 124, the acquisition unit 126, and the determination unit 128 may be realized by an integrated circuit such as an application specific integrated circuit (ASIC) or a field-programmable gate array (FPGA). Furthermore, at least a part of the control unit 124, the acquisition unit 126, and the determination unit 128 may be configured by an electronic circuit including a discrete device.

The control unit 124 outputs various operation signals, and controls the operation of each pump 76, the operation of each clamp 78, and the operation of the reactor driving unit 80. The acquisition unit 126 acquires the amount of received transmitted light (or the amount of received scattered light) over time on the basis of the electrical signal output from the sensor unit 28. The determination unit 128 performs various determinations.

The storage unit 122 can include a volatile memory (not illustrated) and a non-volatile memory (not illustrated). Examples of the volatile memory include a random access memory (RAM) and the like. The volatile memory is used as a working memory of the processor, and temporarily stores data and the like necessary for processing or operation. Examples of the non-volatile memory include a read only memory (ROM) and a flash memory. The non-volatile memory is used as a memory for storage, and stores a program, a table, a map, and the like. At least a part of the storage unit 122 may be provided in the processor, the integrated circuit, or the like as described above.

The pump drive circuit 114 supplies power to the actuator of the pump 76 to be operated according to a pump operation signal output from the controller 112. Each clamp drive circuit 116 supplies power to the actuator of the clamp 78 to be operated according to the clamp operation signal output from the controller 112. The reactor drive circuit 118 supplies power to the actuator of the reactor driving unit 80 in response to a reactor operation signal output from the controller 112.

### [2 Cell Culture Processing]

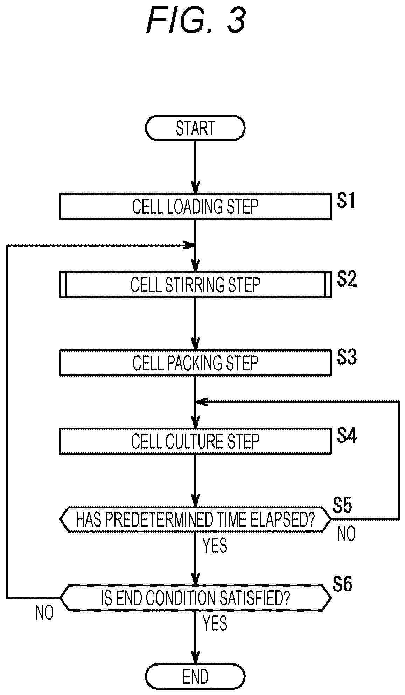

Fig. 3 is a flowchart of cell culture processing. The user uses the input apparatus to perform an operation of starting the cell culture processing. The calculation unit 120 starts the cell culture processing illustrated in Fig. 3 in response to the start operation.

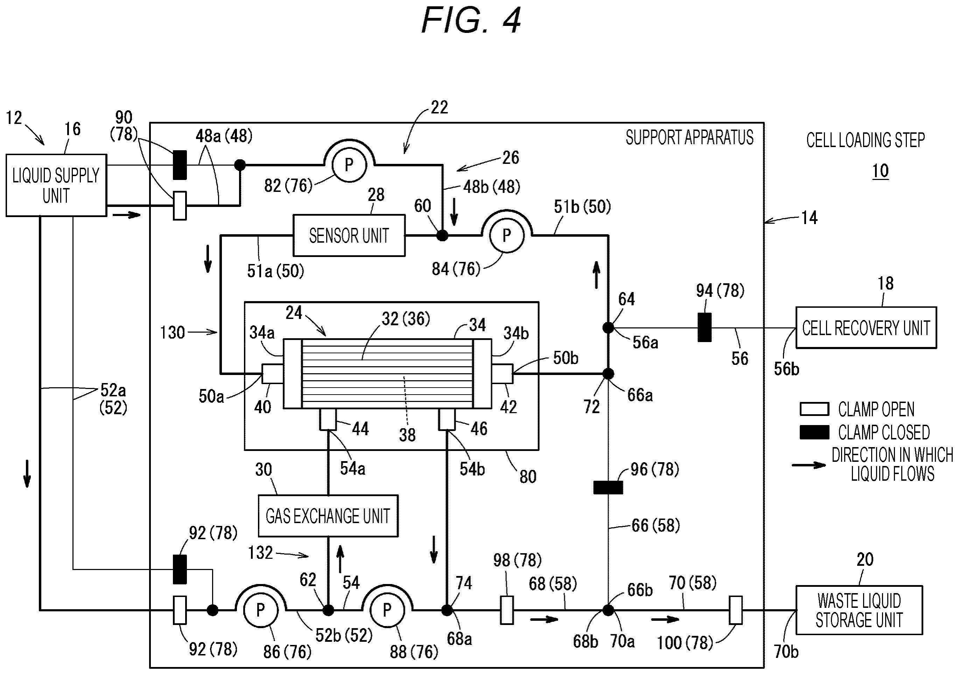

In step S1, the calculation unit 120 performs a cell loading step. The control unit 124 outputs a pump operation signal corresponding to the cell loading step to the pump drive circuit 114. In addition, the control unit 124 outputs a clamp operation signal corresponding to the cell loading step to the clamp drive circuit 116. Then, the cell culture apparatus 10 enters the state illustrated in Fig. 4. The cell suspension filled in the medical bag of the liquid supply unit 16 flows through the first supply flow path 48 and the first circulation flow path 50 and is supplied to the bioreactor 24. Thus, the cells are introduced into the first region 36 (inside the hollow fiber membrane 32) of the bioreactor 24. The control unit 124 stops the cell loading step at a predetermined timing. When step S1 ends, the processing proceeds to step S2.

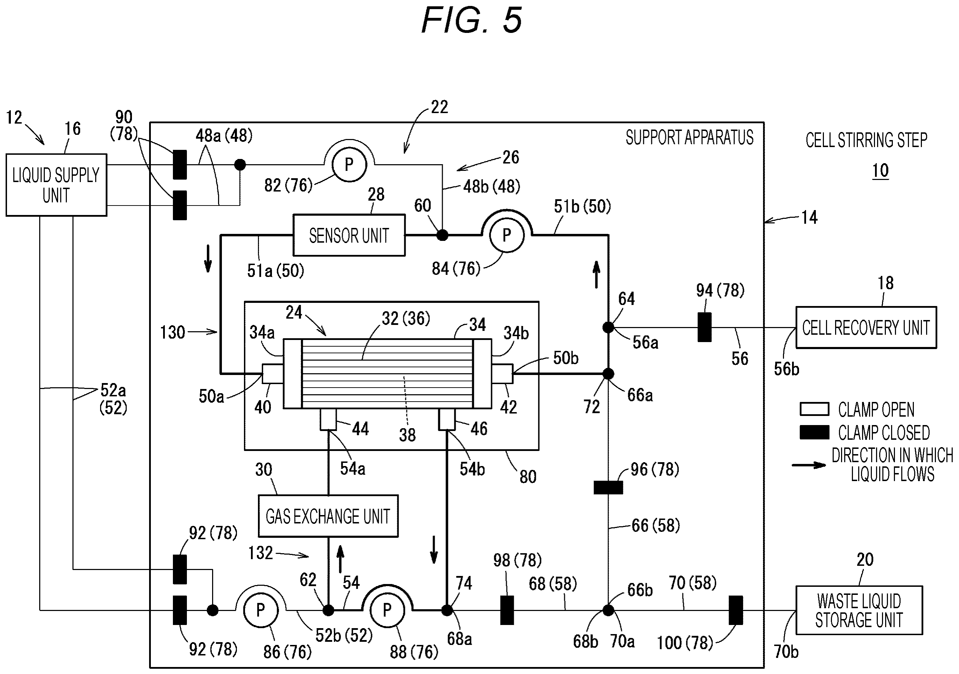

In step S2, the calculation unit 120 performs a cell stirring step. The control unit 124 outputs a pump operation signal corresponding to the cell stirring step to the pump drive circuit 114. In addition, the control unit 124 outputs a clamp operation signal corresponding to the cell stirring step to the clamp drive circuit 116. Then, the cell culture apparatus 10 enters the state illustrated in Fig. 5. Details of the cell stirring step will be described later. In the cell stirring step, the cell suspension is stirred and the cells are diffused into the cell suspension. A state in which cells are uniformly diffused in the cell suspension is referred to as a uniform diffusion state. In a uniform diffusion state, nutrients are evenly distributed among the cells. Therefore, the uniform diffusion state can increase the growth rate of the cells. In addition, it is possible to increase the survival rate of cells by setting the state to the uniform diffusion state. When step S2 ends, the processing proceeds to step S3.

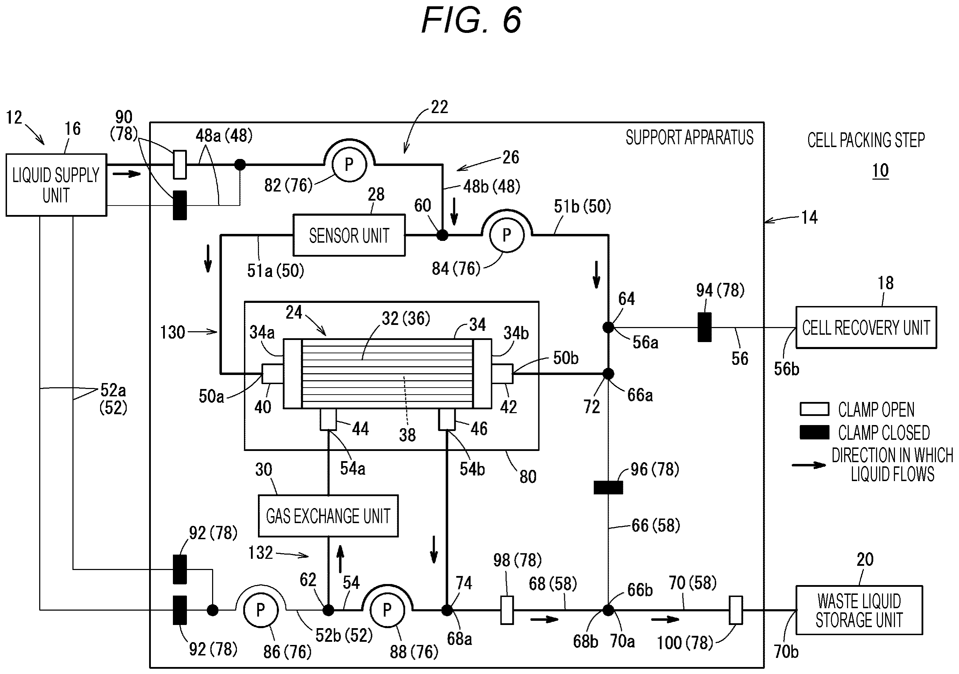

In step S3, the calculation unit 120 performs a cell packing step. The control unit 124 outputs a pump operation signal corresponding to the cell packing step to the pump drive circuit 114. In addition, the control unit 124 outputs a clamp operation signal corresponding to the cell packing step to the clamp drive circuit 116. Then, the cell culture apparatus 10 enters the state illustrated in Fig. 6. The culture medium filled in the medical bag of the liquid supply unit 16 flows through the first supply flow path 48 and the first flow path 51a and is supplied to the bioreactor 24. In addition, the culture medium filled in the medical bag of the liquid supply unit 16 flows through the first supply flow path 48 and the second flow path 51b and is supplied to the bioreactor 24. As a result, the cells remaining in the first circulation flow path 50 move to the first region 36 (inside the hollow fiber membrane 32) of the bioreactor 24. The supply amount of the culture medium from the first flow path 51a to the bioreactor 24 is preferably equal to the supply amount of the culture medium from the second flow path 51b to the bioreactor 24. In addition, the culture medium circulates in the circulation path 132 including the bioreactor 24 and the second circulation flow path 54. The excess culture medium is discharged to the waste liquid storage unit 20. The control unit 124 stops the cell packing step when a predetermined packing time has elapsed from the start of the cell packing step or when the supply amount of the culture medium reaches a predetermined amount. When step S3 ends, the processing proceeds to step S4.

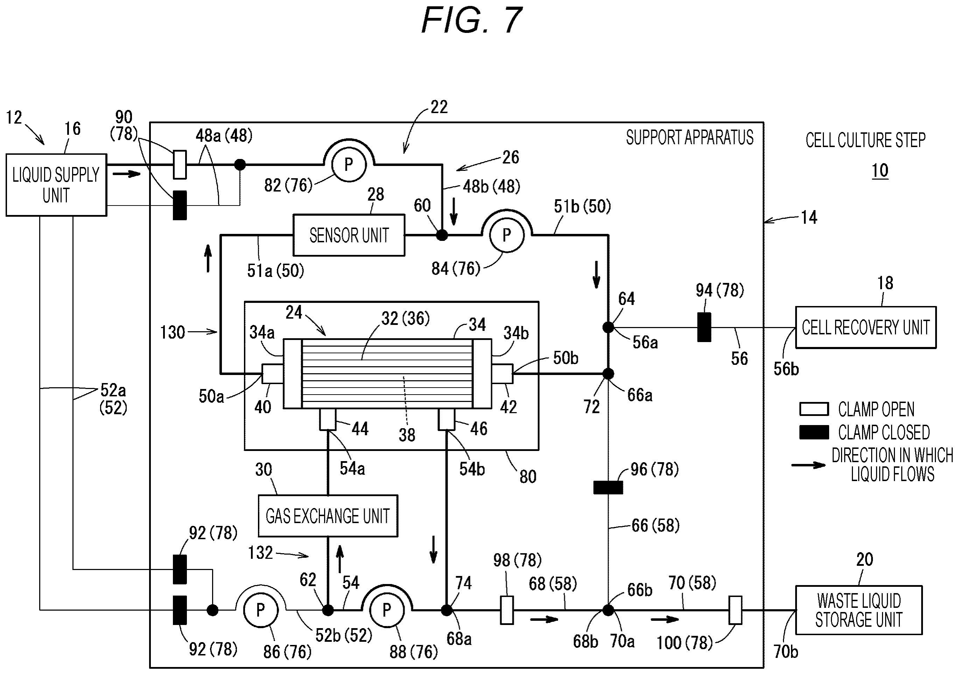

In step S4, the calculation unit 120 performs a cell culture step. Here, the control unit 124 outputs a pump operation signal corresponding to the cell culture step to the pump drive circuit 114. In addition, the control unit 124 outputs a clamp operation signal corresponding to the cell culture step to the clamp drive circuit 116. Then, the cell culture apparatus 10 enters the state illustrated in Fig. 7. The culture medium filled in the medical bag of the liquid supply unit 16 flows through the first supply flow path 48 and the second flow path 51b, and is supplied to the first region 36 (inside the hollow fiber membrane 32) of the bioreactor 24. In addition, the culture medium circulates in the circulation path 130 including the bioreactor 24 and the first circulation flow path 50. This provides nutrients to the cells. In addition, the culture medium circulates in the circulation path 132 including the bioreactor 24 and the second circulation flow path 54. Note that the excess culture medium is discharged to the waste liquid storage unit 20.

In step S5, the determination unit 128 determines whether a predetermined culture time (predetermined time) has elapsed since the start of the cell culture step. The storage unit 122 stores a predetermined time in advance. In a case where the predetermined time has elapsed (step S5: YES), the processing proceeds to step S6. On the other hand, in a case where the predetermined time has not elapsed (step S5: NO), the cell culture step of step S4 is continuously performed.

When the processing proceeds from step S5 to step S6, the determination unit 128 determines whether the end condition of the series of cell culture processing illustrated in Fig. 3 is satisfied. The storage unit 122 stores in advance a calibration curve indicating the relationship between the amount of received transmitted light (or scattered light) of the cell suspension and the number of cells. In addition, the storage unit 122 stores a threshold value as an end condition in advance. The determination unit 128 estimates the number of cells contained in the cell suspension on the basis of the amount of light received by the sensor unit 28 and the calibration curve. The determination unit 128 determines that the end condition is satisfied in a case where the estimated number of cells is equal to or greater than the threshold value. Note that the storage unit 122 may store a calibration curve indicating the relationship between the amount of received transmitted light (or scattered light) of the cell suspension and the cell concentration. In a case where the estimated number of cells is equal to or larger than the threshold value (step S6: YES), the series of cell culture processing is ended. On the other hand, in a case where the estimated number of cells is less than the threshold value (step S6: NO), the processing returns to the cell stirring step of step S2.

When the cell culture processing illustrated in Fig. 3 is completed, the control unit 124 supplies the dissociation solution to the bioreactor 24, and transfers the cells inside the bioreactor 24 to the cell recovery unit 18.

### [3 Cell Stirring Step]

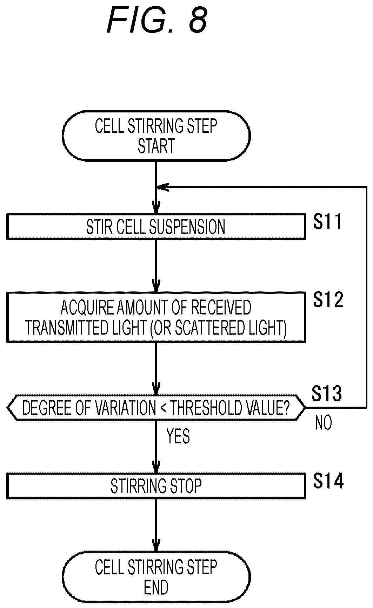

Fig. 8 is a flowchart of the cell stirring step. After step S1 illustrated in Fig. 3 or in a case where the determination is NO in step S6, a cell stirring step described below is performed.

In step S11 (cell stirring step), the control unit 124 stirs the cell suspension in the bioreactor 24. The control unit 124 outputs a pump operation signal for operating the first circulation pump 84 to the pump drive circuit 114. The first circulation pump 84 operates in response to the pump operation signal. As illustrated in Fig. 5, the cell suspension circulates in a circulation path 130 composed of the bioreactor 24 and the first circulation flow path 50. Thereby, the cell suspension is stirred. Further, the control unit 124 may output a reactor operation signal for operating the reactor driving unit 80 to the reactor drive circuit 118. The reactor driving unit 80 alternately rotates the bioreactor 24 in one direction and in the opposite direction according to the reactor operation signal. This allows the cell suspension to be homogeneously diffused more quickly. Note that the processing of step S11 is continued until the processing proceeds to step S14.

In step S12 (acquisition step), the acquisition unit 126 acquires the amount of received transmitted light on the basis of the electric signal output by the sensor unit 28. The acquisition unit 126 may acquire the amount of received scattered light instead of the amount of received transmitted light. The acquisition unit 126 acquires the amount of received transmitted light every predetermined sampling time.

In step S13 (determination step), the determination unit 128 compares the degree of variation of the received light amount of the transmitted light with the threshold value of the degree of variation. For example, the determination unit 128 calculates a standard deviation of the amount of received transmitted light as the degree of variation. The storage unit 122 stores in advance a threshold value of the degree of variation (a threshold value of the standard deviation). As the threshold value, an upper limit value of an allowable degree of variation (standard deviation) is set. The determination unit 128 calculates the standard deviation of the received light amount of the transmitted light on the basis of the received light amount of the transmitted light for the most recent predetermined number of times. The determination unit 128 determines that the cells have reached the uniform diffusion state in the cell suspension on the basis of the fact that the standard deviation is smaller than the threshold value. In a case where the standard deviation is smaller than the threshold value (step S13: YES), the processing proceeds to step S14. On the other hand, in a case where the standard deviation is equal to or larger than the threshold value (step S13: NO), the processing returns to step S11. In this case, the processing of step S11 is continued.

When the process proceeds from step S13 to step S14, the control unit 124 stops stirring the cell suspension. That is, the control unit 124 stops the first circulation pump 84. In addition, the control unit 124 also stops the rotation of the bioreactor 24. When step S14 ends, the processing proceeds to the cell packing step (step S3) illustrated in Fig. 3.

### [4 Effects of Present Embodiment]

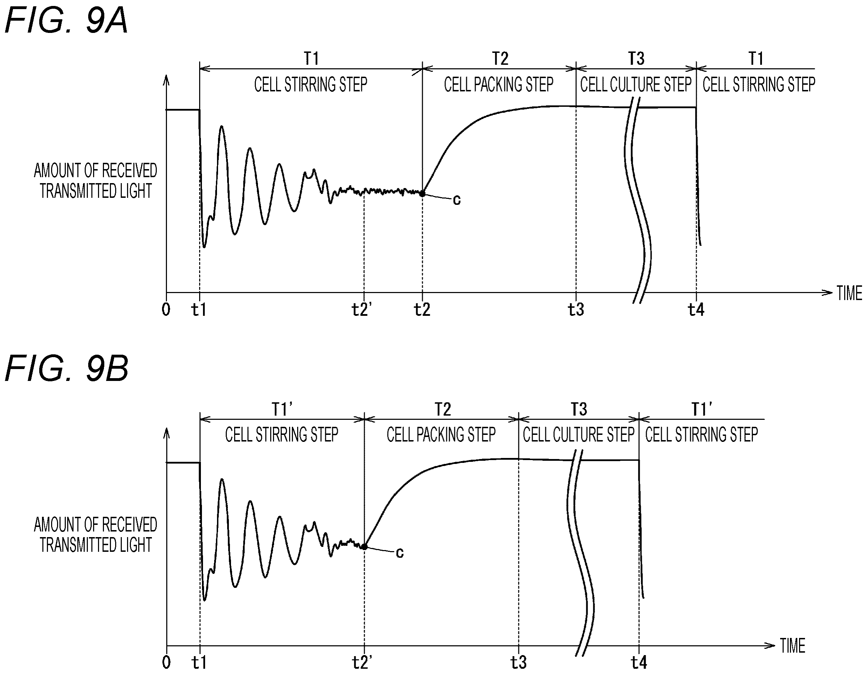

Figs. 9A and 9B are time charts illustrating temporal changes in the amount of received transmitted light detected by the sensor unit 28. Fig. 9A illustrates a temporal change in the amount of received transmitted light in the cell culture processing without using the culture control apparatus 110. Fig. 9B illustrates a temporal change in the amount of received transmitted light in the cell culture processing using the culture control apparatus 110. In the cell culture processing illustrated in Fig. 9A, the time T1 from the time point t1 to the time point t2 is the execution time of the cell stirring step. In the cell culture processing illustrated in Fig. 9A, the time T2 from the time point t2 to the time point t3 is the execution time of the cell packing step. In the cell culture processing illustrated in Fig. 9A, the time T3 from the time point t3 to the time point t4 is the execution time of the cell culture step.

In the cell stirring step illustrated in Fig. 9A, most of the cells are inside the bioreactor 24 at a stage before stirring (before time point t1). Therefore, the culture medium flowing through the first circulation flow path 50 contains few cells. Therefore, the turbidity of the liquid flowing through the first circulation flow path 50 is low, and the amount of received transmitted light detected by the sensor unit 28 is large. When stirring is started at time point t1, cells inside the bioreactor 24 flow through the first circulation flow path 50. Then, a portion having a high cell concentration and a portion having a low cell concentration alternately appear in the liquid flowing through the first circulation flow path 50. As a result, the amount of received transmitted light detected by the sensor unit 28 changes with the lapse of time. When the stirring is continued, the received light amount of the transmitted light detected by the sensor unit 28 gradually converges to a substantially constant value c. The fact that the received light amount of the transmitted light detected by the sensor unit 28 converges to the constant value c means that the cells are uniformly diffused in the liquid (cell suspension). The cell stirring step illustrated in Fig. 9B has already converged to the constant value c at a time point t2' earlier than the time point t2. That is, the cell stirring step performed from the time point t2' to the time point t2 is unnecessary.

In the present embodiment, the determination unit 128 compares the standard deviation (degree of variation) with a threshold value (predetermined degree) (step S13 in Fig. 8). As a result, the determination unit 128 can determine that the uniform diffusion state has been reached. According to the present embodiment, the cell stirring step can be executed only for a necessary minimum time. According to the present embodiment, the execution time (time T1') of the cell stirring step can be shortened, and the execution time of the cell culture processing illustrated in Fig. 3 can be shortened. Therefore, according to the present embodiment, it is possible to contribute to the optimization of the cell culture processing.

Note that, the present invention is not limited to the above disclosure, and various configurations can be adopted without departing from the gist of the present invention.

## Claims

1. A stirring method comprising:
a cell stirring step of stirring cell suspension by circulating the cell suspension in a circulation path including a bioreactor;
an acquisition step of acquiring a predetermined physical quantity related to the cell suspension at a measurement target site in the circulation path over time; and
a determination step of determining that a uniform diffusion state in which cells are uniformly diffused in the cell suspension is reached on a basis of a fact that a degree of variation of the physical quantity is smaller than a predetermined degree.

2. The stirring method according to claim 1, wherein
the degree of variation of the physical quantity is grasped on a basis of a standard deviation of a plurality of the physical quantity within a predetermined unit time.

3. The stirring method according to claim 1, wherein
the physical quantity is acquired using an optical sensor.

4. The stirring method according to claim 3, wherein
the physical quantity is at least one of an amount of transmitted light transmitted through the cell suspension and an amount of scattered light scattered by the cell suspension.

5. The stirring method according to claim 1, wherein
in the cell stirring step, stirring of the cell suspension is stopped in a case where it is determined in the determination step that the uniform diffusion state is reached.

6. The stirring method according to any one of claims 1 to 5, wherein
in the cell stirring step, the cell suspension is stirred by rotating the bioreactor using an actuator.

7. A stirring apparatus comprising:
a control unit that stirs cell suspension by circulating the cell suspension in a circulation path including a bioreactor;
an acquisition unit that acquires a predetermined physical quantity related to the cell suspension at a measurement target site in the circulation path over time; and
a determination unit that determines that a uniform diffusion state in which cells are uniformly diffused in the cell suspension is reached on a basis of a fact that a degree of variation of the physical quantity is smaller than a predetermined degree.

8. The stirring apparatus according to claim 7, wherein
the degree of variation of the physical quantity is grasped on a basis of a standard deviation of a plurality of the physical quantity within a predetermined unit time.

9. The stirring apparatus according to claim 7, wherein
the physical quantity is acquired using an optical sensor.

10. The stirring apparatus according to claim 9, wherein
the physical quantity is at least one of an amount of transmitted light transmitted through the cell suspension and an amount of scattered light scattered by the cell suspension.

11. The stirring apparatus according to claim 7, wherein
the control unit stops stirring of the cell suspension in a case where the determination unit determines that the uniform diffusion state is reached.

12. The stirring apparatus according to any one of claims 7 to 11, wherein
the control unit stirs the cell suspension by rotating the bioreactor using an actuator.
